(19) **Europäisches Patentamt / European Patent Office / Office européen des brevets**

(11) **EP 3 025 717 B1**

(12) **EUROPEAN PATENT SPECIFICATION**

(45) Date of publication and mention
of the grant of the patent:
**07.11.2018 Bulletin 2018/45**

(51) Int Cl.:
***A61K 31/5415*** *(2006.01)*    ***A61K 31/455*** *(2006.01)*
***A61P 29/00*** *(2006.01)*

(21) Application number: **13885633.1**

(22) Date of filing: **27.05.2013**

(86) International application number:
**PCT/MX2013/000070**

(87) International publication number:
**WO 2014/193210 (04.12.2014 Gazette 2014/49)**

(54) **SYNERGISTIC PHARMACEUTICAL COMPOSITION OF TWO ANALGESICS HAVING A DISTINCT PHARMACOKINETIC PROFILE**

SYNERGISTISCHE PHARMAZEUTISCHE ZUSAMMENSETZUNG AUS ZWEI ANALGETIKA MIT UNTERSCHIEDLICHEM PHARMAKOKINETIKPROFIL

COMPOSITION PHARMACEUTIQUE SYNERGIQUE DE DEUX ANALGÉSIQUES PRÉSENTANT UN PROFIL PHARMACOCINÉTIQUE DISTINCT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.06.2016 Bulletin 2016/22**

(73) Proprietor: **FARMACÉUTICOS RAYERE, S.A.**
**03300, Distrito Federal (MX)**

(72) Inventors:
• **AGUILERA SUÁREZ, Graciela de los Ángeles**
**C.P. 03300 Distrito Federal (MX)**
• **JUÁREZ LORA, Martha Rosaura**
**C.P. 03300 Distrito Federal (MX)**
• **GÓMEZ SÁNCHEZ, Carmen Miguel**
**C.P. 03300 Distrito Federal (MX)**

(74) Representative: **Carlos Hernando, Borja**
**Garrigues IP, S.L.P.**
**Hermosilla, 3**
**28001 Madrid (ES)**

(56) References cited:
**EP-A2- 0 492 747**    **WO-A1-2007/083985**
**WO-A1-2007/148950**    **WO-A1-2012/176155**
**US-A- 3 973 027**    **US-A1- 2004 229 038**
**US-A1- 2007 281 927**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Summary of the invention**

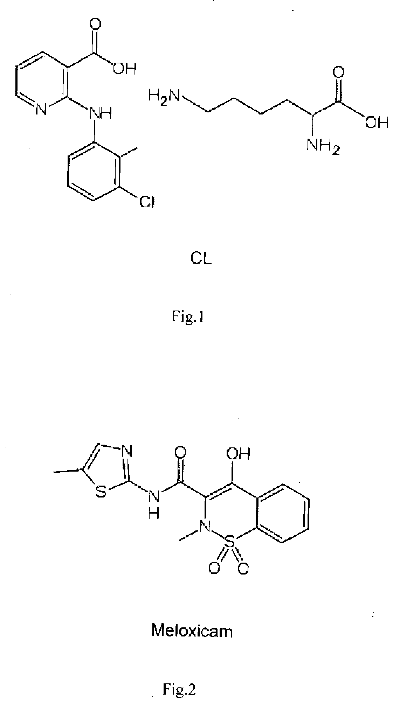

The scope of the invention is defined by the appendent claims. The present invention relates to pharmaceutical compositions containing two active principles of the group known as non-steroidal anti-inflammatory analgesics (NSAIDs), one being lysine clonixinate (CL) and the other; meloxicam (MXC). Lysine clonixinate referred to in the present invention is the lysine salt of 2-[(3-chloro-2-methylphenyl)amino]-3-pyridinecarboxylic acid and belongs to the group of nicotinic acid derivatives (Fig. 1). Meloxicam referred to in the present invention is the compound named 4-hydroxy-2-methyl-*N*-(5-methyl-2-thiazolyl)-2*H*-1,2-benzothiazine-3-carboxamide-1,1-dioxide and belongs to the group of enolic acids (oxicams) (Fig. 2).

According to its pharmacokinetics, the plasma levels of CL indicate that this is a rapid onset drug (Tmax of 0.84 to 0.44 h) and short duration (t½ of 1 to 1.5 h), (Marcelin G, Angeles C, Garda A, et al. 2010). Unlike CL, MXC is a slow onset drug (Tmax of 5 to 6 h) and prolonged duration (t½ of 15 to 20 h) (Marcelin G, Hernandez J. Angeles C, et al. 2005). When lysine clonixinate is combined with meloxicam (CL-MXC), in specific proportions, the combination produces analgesic pharmacological effects which indicate a super-additivity (synergy). Furthermore the anti-inflammatory effect is three times higher than that resulting from the separate administration of the drugs. This allows reducing the therapeutic dose of the drugs when they are administered separately.

Owing to the characteristics of the drugs of which it is composed, the CL-MXC combination is designed for the treatment of inflammation and moderate to severe acute pain of the musculoskeletal system, for the treatment of post-operative pain and dental pain, and for migraine and headache. The adverse effect profile observed after administration of the CL-MXC combination suggests that this therapeutic strategy may be a promising tool in the relief of moderate to severe acute pain and the inflammatory processes that accompany it, with the advantage of relieving pain with a high efficiency.

**Background of the invention**

Painful processes involve the participation of different peripheral and central mechanisms, as well as numerous substances and receptors that are responsible for transmission and modulation of the pain response. Sometimes analgesic alone may not be effective for the treatment of acute pain. When this occurs drug combinations may be used. This can be achieved by blocking the pain pathways at different levels simultaneously and/or increasing the range of action, combining an analgesic of rapid onset and short action with one of slow onset and prolonged duration. Similarly when a combination is used, the additive and synergistic effects of the different drugs make possible the use of lower doses, which, at the same time, allows the reduction of adverse effects (Barkin R, 2001).

The strategy of using combinations of drugs for the treatment of pain was proposed in 1986 by the World Health Organization (WHO, 1986). The WHO proposal recognised that most cases of cancer pain could be relieved by using a simple medical treatment based on what is called "the analgesic stairs of 3 steps", which, depending on the severity of the patient's pain, recommends the use of various types of analgesics also in combination with adjuvant drugs when necessary. The reasoning behind the combined analgesic therapy is that the blocking of pain pathways at different levels can be achieved simultaneously. The time interval of therapeutic effect can also be increased by combining analgesics of rapid onset and short action with analgesics of slow onset and prolonged duration that generally have different action mechanisms.

The term "balanced analgesia" was first used at the beginning of the nineties from strategies used to achieve a better solution in the treatment of pain in the post-operative period (Dahl J, Rosenberg J, Dirkes W, et al. 1990). The studies, in which analgesics with different action mechanisms were administered together, demonstrated that the use of this type of analgesia produces an additive or synergistic effect between the different analgesics employed, with a concomitant reduction in adverse effects due to a reduction in the doses used for each drug (Kelhet H, Wemer M and Perkins F, 1999).

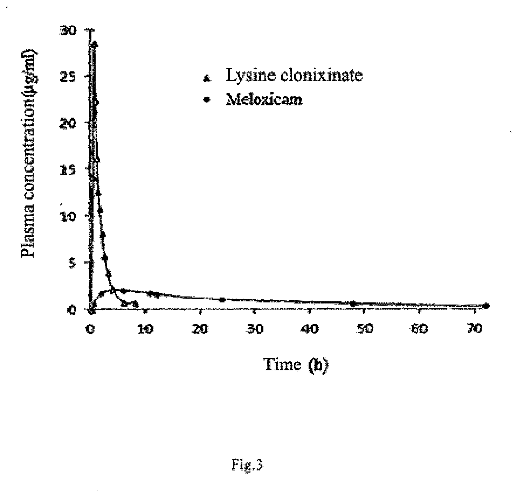

In the present invention a combination of the two drugs was achieved: clonixinate lysine, an analgesic of rapid onset and short action, and meloxicam, of slow onset and prolonged duration (Fig. 3), such that the therapeutic analgesic potential of each was increased separately.

**NSAIDs**

The main activity of NSAIDs is the inhibition of the activity of prostaglandin-endoperoxide G/H synthase enzymes, also known as cyclooxygenase (COX), and of the lipoxygenase enzyme (LOX), in their different isoforms: COX-1, COX-2, COX-3 and LOX-5, LOX-12 and LOX-15 respectively (Franchi A, Di Girolamo G, Farina M, et al. 2000). Overall, the COX and the LOX isoforms are part of the enzyme complex involved fundamentally in the body's inflammatory response and in the repair of damaged tissue (Gronert K, 2008). Both enzyme families metabolise, by different routes, to arachidonic acid (AA) released from the cell membrane to produce molecules generically known as prostanoids: Prostaglandins (PGE2, PGD$_2$, PGF$_{2a}$), prostacyclin (PGI2, PGI3), thromboxane (TXA$_2$, TXB$_2$); leukotrienes (LTA$_4$, LTB$_4$, LT$_4$, LTD$_4$) and lipoxins (LXA4 and LXB$_4$). Eicosatrienoic acids generated by the cytochrome-P450 epoxygenase (CYP) enzyme activity are also produced by arachidonic acid derivatives. All these molecules derived from arachidonic acid have local activities that implement a complex physiological control over many body systems, also taking part in pathological processes mainly of inflammation and immune response, and as messengers in the central nervous system.

The enzymatic activity of COX-1 is mainly expressed in a constitutive way in most cells, whereas the expression of COX-2 is mainly induced by mediators for inflammation and cancer, such as cytokines and growth factors. Traditionally it was thought that the functions of COX-1 were only of physiological type such as the cytoprotection of the gastric epithelium, while those of COX-2 were exclusively related to inflammatory and immunological response. However, subsequent studies showed that both COX isoforms are involved both in physiological and pathological processes in the body, for example the activity of COX-2 is essential for the production of vascular prostacyclin and also prostanoids that are produced by the action of COX -2 are involved in the normal development and function of the kidney (Gronert K, 2008; Ong C, Lirk P et al. 2007). In turn, the products of metabolism of lipoxygenase, leukotrienes and lipoxins, epilipoxins and hepoxlins, also have an important role in inflammation and are also involved in anaphylaxis (Vandana R, 2007). It is generally considered that the biological action of prostanoids is complex because a single molecule may have different functions, some of which are counter active to each other and depend on the cell type, nature of the stimulatory response and the type of receptor.

The relief of pain and inflammation is undoubtedly the main cause for the therapeutic indication of COX inhibitors. For over three decades and to date, the involvement of prostanoids and their receptors in pain generation has been an area of intense research. It is known that these molecules are mediators of immune and inflammatory responses, since its administration reproduces the major signs of inflammation including hyperalgesia. Some prostanoids contribute to the generation of pain by acting both on a central and peripheral level. Indirectly, they sensitise the nociceptors, particularly PGE2 and PGI2, producing a strong sensitisation activating the EP and IP receptors respectively in peripheral nerve terminals (Samad T, Sapirstein A. and Woolf C, 2002; Tilley S, Coffinan T and Koller B, 2001; Zeilhofer H 2007).

In the central nervous system, one of the critical pathways for the generation of hypersensity to pain involves the induction of COX-2 in neurons of the dorsal horn of the spinal cord (Latremolier A and Wolf C, 2009).

Since they have such varied activities in different organs, the inhibition of COX enzyme isoforms results in the NSAIDs, as well as suppressing the inflammatory response and pain, being associated to a large number of adverse effects. The type and severity of the adverse effects depend on the inhibitory ratio to COX-1 or COX-2 which is characteristic of each NSAID. The NSAIDs' adverse effects include alterations in kidney function, effects on blood pressure, liver damage and platelet inhibition. The administration of NSAIDs to patients with disorders of haemostasis or in perioperative states increases the risk of bleeding through the inhibition of platelet functions, which can result in increased bleeding (Sagripanti A, Ferdeghini M, Pinori E et al., 1989).

In clinics, the most often observed adverse effects of NSAIDs with unspecified inhibitory activity on COX-1 and COX-2 are gastrointestinal effects. In turn, several clinical studies have shown an increased risk of cardiovascular adverse effects in patients taking specific inhibitors of COX-2. In several studies carried out in vitro, the inhibitory activity of various NSAIDs has been determined for both COX-1 and COX-2, the concentration of NSAIDs being obtained which inhibits 50% (CI$_{50}$) of the activity of each of these isoforms and as well as their ratio: CI$_{50}$COX-2/CI$_{50}$COX-1 (Mitchell J, Akarasereenont P, Thiemermann C. et. al. 1994; Pallapies D, Salinger A, Meyer zum Gottesberge A et al. 1995; Sagripinti A, Ferdeghini M, Pinori E et al., 1989). One of the highest ratio values CI$_{50}$COX-2/CI$_{50}$COX-1 found, is aspirin because it inhibits 166 times more the constitutive COX-1 than the COX-2. As is well known, acetylsalicylic acid is an NSAID with high gastrointestinal irritability and inhibitory effect on platelet aggregation.

Even when the ratio CI$_{50}$COX-2/CI$_{50}$COX-1 has been used to get an idea of inhibitory selectivity of analgesics for COX-2 or COX-1, the NSAIDS activity largely depends on the expression of both isoforms on each tissue and on whether this is intact or in a state of inflammation. In this regard, it has been found that neither lysine clonixinate, nor meloxicam cause adverse effects in the gastrointestinal tract or platelet function as severely as do other NSAIDs (Hawkey C, Kahan A, Steinbruck K et al. 1998; Dequeker J, Hawkey C, Kahan A et al. 1998; Kramer E, Sassetti B, Kaminker A et al. 2001).

**Lysine clonixinate**

The Lysine clonxinate used in the present invention has the chemical name: lysine salt of 2-[(3-chloro-2-methylphenyl)amino]-3-pyridinecarboxylic acid, represented by formula $C_{13}H_{11}ClN_2O_2.C_6H_{14}N_2O_2$. Its structural formula is shown in Figure 1. It is an analgesic classified in the group of non-steroidal anti-inflammatory drugs (NSAIDs) derived from nicotinic acid. Like other NSAIDs, CL reversibly inhibits the activity of COX-1 and COX-2, having also been shown the inhibitory action of leukotriene synthesis through the inhibition of lipoxygenase (Franchi A, Di Girolamo G, Farina M et al. 2000). The anti-prostaglandinic analgesic action of CL occurs due to the role of the $PGE_2$ and $PGI_2$ by sensitising the nerve terminals to the irritative action of serotonin, bradykinin, etc. Also, as mentioned, as an inhibitor of COX-2, the analgesic effect can also occur at the central nervous system level (Latremolier A and Wolf C, 2009).

In addition to its inhibitory effect on prostaglandin synthesis, other action mechanisms to explain the efficacy of this powerful NSAID have been proposed. For example, another effect of CL that has been studied is the inhibition of nitric oxide synthesis through inhibition of the NO synthetase (Di Girolamo G, Farina M, Ribeiro M et al. 2003). The production of nitric oxide (NO) plays an important role in inflammation, in these circumstances the production of the NO synthase enzyme is induced and the NO produced becomes part of the response of the body's defence and tissue repair.

NO is also synthesized in neurons of the dorsal root of the spinal cord and its role in the pain process has been implicated in the mechanisms of central nervous system sensitisation (Latremolier A and Wolf C 2009). Also, it has been disclosed that CL has an indirect analgesic action at the central nervous system level, since it has been found in studies in rats that it regulates receptors $\mu$ and k, in rat brains (Orti E, Coirini H y Pico J. 1989).

CL is indicated for the relief of headaches, muscle and joint pains, neuritic algias, toothache, earache, dysmenorrhoea, post-traumatic or post-surgical pain. Its analgesic activity is 20 times more intense than that of acetylsalicylic acid (Ferrari P, 1976). CL is effective in the management of acute pain, and is used particularly in the post-operative period. Its pharmacological effectiveness is recognised for the treatment of moderate to severe pain syndromes such as headaches, muscle pain, joint pain, neuritic pain; toothache, earache, dysmenorrhoea, post-traumatic or post-surgical pain and even in the treatment of migraine (Krymchantowski A, Barbosa J, Cheim C et al. 2001).

One of the main advantages of CL is that, due to its physical-chemical properties, it is rapidly absorbed through the gastrointestinal tract. The values of the pharmacokinetic parameters after an oral dose of 250 mg of CL, are the following: maximum plasma concentration (Cmax): $32.39 \pm 8.32$ $\mu$g/ml; time to the peak of maximum concentration (Tmax): $0.64 \pm 0.2$ h; area under the curve from 0 to 8 hours (AUC 0-8h): $48.92 \pm 16.51$ $\mu$gh/ml); half-life time (t½): $1.3 \pm 024$ h; clearance (CL): $5.64 \pm 1.99$ l/h and apparent volume of distribution (Vd): $10.22 \pm 2.91$ (Marcelin G Angeles C, Garcia A et al. 2010).

The concentrations in serum of CL are very similar when administered intravenously or orally after 45 minutes, and the areas under the respective curve are also very similar, demonstrating the high degree of bio-availability by the oral route. These pharmacokinetic characteristics make the analgesic response to CL almost immediate.

Although it has a powerful analgesic effect, the CL does not depress the bone marrow nor interfere with clotting factors, so it does not alter the number nor the platelet function (Kramer E, Sassetti B, Kaminker A et al. 2001). Being a non-narcotic analgesic, it does not alter patients' vital signs nor the state of consciousness. In a comparative study in rats, which were administered 2.5, 10 and 30 mg/kg lysine clonixinate, or 1 mg/kg of ketorolac tromethamine by oral route, the release of prostanoids in the brain, gastric mucosa, lung and kidney incubated ex vivo was studied (Pallapies D, Salinger A, Meyer zum Gottesberge A et al, 1995). In all the organs investigated it was found that both drugs inhibit cyclooxygenase (COX) in a dosage-dependent way, although ketorolac was stronger and had a more durable effect than lysine clonixinate.

**Meloxicam**

Meloxicam (MXC) is an NSAID which belongs to the group of enolic acid derivatives "oxicams". Its chemical name is 4-Hydroxy-2-methyl-N- (5-methyl-2-thiazolyl)-2H-1,2-benzothiazine-3-carboxamide-1,1-dioxide. It is represented by the formula $C_{14}H_{13}N_3O_4S_2$. Its structural formula is shown in Figure 2. Like other NSAIDs, meloxicam inhibits the enzyme cyclooxygenase (COX-1 and COX-2), so that the effect of meloxicam is the reduction of pain by inhibiting the synthesis of prostaglandins, thromboxanes and prostacyclin. Meloxicam has been considered a NSAID of second or third generation, with preferential inhibitory activity towards COX-2 (Lazer E, Miao C, Cywin C et al. 1996).

In preclinical studies, it has shown the anti-inflammatory, anti-pyretic and analgesic effect in a variety of animal models (Engelhardt G, Homma D, K Schlegel et al. 1995). In the model of oedema induction through carrageen in rats, meloxicam proved to have a greater anti-inflammatory action than piroxicam, indomethacin and diclofenac, as well as demonstrating a prolonged and powerful inhibition of somatic pain. In the model of arthritis induction in rats, meloxicam was a more powerful inhibitor than piroxicam, indomethacin, diclofenac and naproxen.

In a study to determine the effect on human gastric mucosa of meloxicam compared to indomethacin, pieces of human gastric mucosa were incubated to determine its inhibitory activity on the synthesis of prostanoids. The results showed that meloxicam is a less potent inhibitor of eicosanoid synthesis in gastric mucosa compared to indomethacin, showing a difference in $CI_{50}$ prostaglandin E (PGE), of 11.8 and 1.8 $\mu$M, respectively (Tavares I. 2000). This favours that meloxicam has good gastrointestinal tolerability.

In a study made on human chondrocytes in vitro isolated from healthy articular cartilage, the effect of anti-inflammatory drugs on the activity of COX-1 and COX-2 in resting conditions and in stimulation with EL-1 was studied. (Blanco F, Guitian R, Moreno J et al. 1999). The drugs studied were: meloxicam, dexamethasone, diclofenac, indomethacin and acetylsalicylic acid. Among them, meloxicam proved to be the NSAID with the best inhibitory ratio COX-2/COX-1 (r = 0.12). In studies in patients with osteoarthritis, MXC showed enhanced inhibitory selectivity for COX-2, with a significant reduction in adverse effects compared to those found with the use of diclofenac or piroxicam, with the same efficacy and safety (Dequeker J, Hawkey C, Kahan A et al. 1998).

Meloxicam is administered orally, but can also be administered intramuscularly or rectally. Unlike CL, after oral administration of MXC, absorption is slow, reaching the highest plasma concentrations in 4 - 5 hours, however the absolute bio-availability is 90%. The drug undergoes gastrointestinal recirculation, as a second peak of maximum concentration is observed in 12-14 hours. Drug absorption is unaffected by the presence of food, but it does increase the maximum plasma concentrations by 22 %. The drug is extensively bound to plasma proteins (99%), particularly albumin. In synovial fluid the plasma concentrations observed are of 40-50%, but due to the lower albumin content in synovial fluid, the free fraction of the drug is greater. Meloxicam is extensively metabolised by the CYP2C9 enzyme system (with a smaller contribution of CYP3A4), yielding 4 inactive metabolites. 43% of the administered dose is excreted in the urine, mostly as metabolites, while the remainder is excreted in the faeces. The percentage of native drug in the urine and faeces is 0.2% and 1.6%, respectively (Gates B, Nguyen T, Setter S et al. 2005).

Meloxicam exhibits a linear pharmacokinetic profile with a half-life of elimination of 15 to 20 hours. The steady state is reached after five doses (once-daily). In women, meloxicam plasma concentrations are lower compared to those of men of the same age. At steady state, the half-life time of elimination is 17.9 hours in women and 21.4 hours in men (Marcelin G, Hernandez J. Angeles C et al. 2005). However, maximum concentrations are similar for both sexes.

Compared with traditional NSAIDs, meloxicam does not inhibit platelet aggregation induced by collagen or arachidonic acid. Greater gastric tolerability of meloxicam compared with indomethacin has also been demonstrated, which is related to a relatively low inhibition of COX-1 in the synthesis of PGE in the gastric mucosa (Tavares I, 2000).

US 2007/281927 A1, describes different methods of achieving oral formulations of meloxicam which can improve the absorption thereof and thus obtain maximum plasma concentration of meloxicam in less time than that obtained with the reference commercial formulation.

US Patent 3,973,027, discloses 2 methods of obtaining the drug lysine clonixinate (compound I, PM 408.8). This prior art discloses a method for inducing analgesia with the active ingredient lysine clonixinate in two pharmaceutical forms, oral and injectable solution, no combination with another active ingredient is mentioned.

EP 0492747 A2, discloses pharmaceutical preparations for topical application in the form of a lysine clonixinate cream and gel. Using these topical preparations, the percentage of anti-inflammatory effect as well as the transdermal absorption at different times is described, and these variables are compared using the same type of topical preparations formulated separately with diclofenac.

WO2007/083985 describes the combination lysine clonixinate /diclofenac and WO2007/148950 describes a lysine clonixinate/ketorolac combination.

WO 2012/176155 A1, describes a combination of ketorolac and meloxicam or piroxicam. The application relates to formulations for topical use such as cream, gels, ointments, pastes, etc. that show analgesic and anti-inflammatory effects.

US 2004/229038 A1, discloses meloxicam compositions having a particle size of less than 2000 nm, with the purpose of improving the bioavailability of meloxicam given its low solubility.

**Lysine clonixinate/meloxicam (CL-MXC)**

Considering all the above background, the idea of combining meloxicam: lysine clonixinate (MXC:CL) in a weight ratio of from 1:1 to 1:50 aims to obtain a drug with a high analgesic efficacy with immediate action The synergy between the two can be achieved in the combination, amongst other reasons, by this composition being composed of one analgesic of rapid onset and short action, and one of slow onset and prolonged duration respectively (Fig. 3). Through its composition, another objective in making the CL-MXC combination is to raise the anti-inflammatory effect. In general, in improving therapeutic efficacy, in the formulation of CL-MXC lower amounts of each drug can be used than used clinically and with fewer adverse effects.

*Analgesic efficacy of CL-MXC*

To demonstrate the idea underlying the present invention, various preclinical studies were conducted using CL and MXC to investigate the possible analgesic synergy of the combination. Therefore, the primary objective of this study was to determine the proportion of CL-MXC combination that had the greater analgesic efficacy in the experimental model of formalin in mice. Subsequently, the type and magnitude of the analgesic interaction between meloxicam and lysine clonixinate was determined by isobolographic analysis.

*Animals:* ICR male mice were used being 6-8 weeks old, weighing 20-30 g, which were purchased from the company Harlan Mexico, S.A de C.V. The animals were kept in boxes with food and water *ad libitum* and light-dark cycles of 12 x 12 h until the time of the experiment. The duration of the experiment was always as short as possible, considering that the number of animals used was the minimum necessary. Each animal was used for a single experiment and sacrificed immediately after it, following the ethical guidelines for pain research in experimental animals (Zimmermann M. 1983).

*Drugs and reagents:* MXC and CL were used as raw material provided by Farmacéuticos Rayere S.A. The reagent grade formaldehyde was purchased (Sigma Chemical SA) while the 0.9 % sterile saline solution was of pharmaceutical grade and was purchased from commercial sources. After assessing the solubility in various solvent systems, it has been established that the drugs can be dissolved in still mineral water for human consumption (pH = 7.5), therefore, they were administered (10 ml/kg, orally). In the case of formalin, it was prepared at a concentration of 3% in saline solution (0.9%).

*Experimental model of formalin administration to evaluate the analgesic response to acute inflammatory pain:* The formalin model is a model of acute inflammatory pain, consisting of subcutaneous administration of formalin into the dorsal area of the right hind paw of the mouse and observation of its subsequent behaviour (Dubuisson and Dennis, 1977). Transparent cylinders of 20 cm diameter X 40 cm high were used, with mirrors. At the start of the experiment, the mice were placed in cylinders for a period of 60 minutes to settle. After this time, the mice were removed for the administration of 20 $\mu$L formalin (3% formaldehyde solution). Immediately after formalin administration, the mouse was returned to the cylinder for observing the characteristic behaviour, which consists in counting the number of licks of the injected paw during 5-minute periods to a total time of 45 minutes.

Different groups were used to characterise the dose-response curve of the analgesics by administering these intraperitoneally 20 minutes before the formalin injection. Doses used for meloxicam were 0.042, 0.063, 0.083 and 0.125 mg/kg and for lysine clonixinate: 2.08, 4.16, 8.33 and 75 mg/kg. Groups of animals were used with an average $n \geq 6$. Physiological saline solution (0.9) was administered intraperitoneally as a control of each experimental set.

The licking time used was recorded in phase 2 or inflammatory pain phase, measured at periods of 15 to 45 minutes after the nociceptive stimulus. The percentage of anti-nociception was plotted according to the dose of both analgesics individually or in combination. The data of the dose-response curve were analysed by a variance analysis (ANOVA), followed by a Tukey test. The dose-response curves were adjusted using a minimum square linear regression, and values of $DE_{30}$ and their standard errors were calculated (Tallarida R 2000).

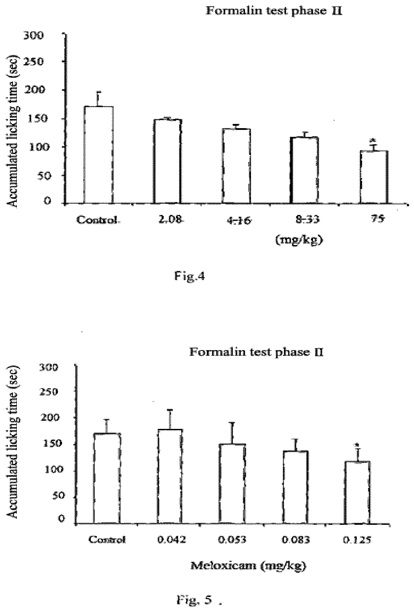

In Figures 3 and 4 the results of the anti-nociceptive response are shown, obtained after intraperitoneal administration of lysine clonixinate and meloxicam respectively and individually. Data are presented as the average (n = 6) $\pm$ e.e; asterisks indicate values of p <0.05 compared to the control group in each case. It can be seen that both drugs progressively reduce the nociceptive effect on increasing the dose, reaching a ceiling effect characteristic of the NSAIDs in this model.

Due to the ceiling effect found, for the isobolographic analysis MXG-CL combination values of the doses were taken that caused the 3 0% of anti-nociceptive effect ($DE_{30}$) of lysine clonixinate and meloxicam (Tallarida, 2000). The $DE_{30}$ for meloxicam was 0.12 $\pm$ 0.004/kg while for lysine clonixinate was 9.80 $\pm$ 1.83 mg/kg, which leads to the estimation in an isobologram of a theoretical additive $DE_{30}$ of the combination (combination dose that only produces a summation effect), of 4.96 $\pm$ 0.92 mg/kg, this was subsequently compared to the experimental dose obtained.

Subsequently the effect of different ratios of combination CL- MXC to select one with a better analgesic response was assessed. The doses used for this assay correspond to those reported as effective in humans, considering that an average human weighs 60 kg. With that reasoning and to determine which ratio of the MXC-CL combination provides greater analgesic efficacy the following ratios were evaluated: 1:16.7, 1:25, 1:33.3, 1:66.7, 1:50 and 1:100 (MXC:CL). In Table 1, the ratios and doses tested in mg/kg are shown.

Table 1.

| Ratio No. | (MXC:CL) | MXC (mg/Kg) | CL (mg/Kg) | Total (mg/Kg) |
|---|---|---|---|---|
| I | 1:16.7 | 0.13 | 2.08 | 2.21 |
| II | 1:25.0 | 0.08 | 2.08 | 2.17 |
| III | 01:33.3 | 0.06 | 2.08 | 2.15 |
| IV | 01:50.0 | 0.04 | 2.08 | 2.13 |
| V | 1:66.7 | 0.06 | 4.17 | 4.23 |
| VI | 1:100 | 0.04 | 4.17 | 4.21 |

As a result of the assessment of the analgesic effect the ratio1:33 was selected as it provided the greatest analgesic effect measured as the decrease in the total accumulated licking time in the mice.

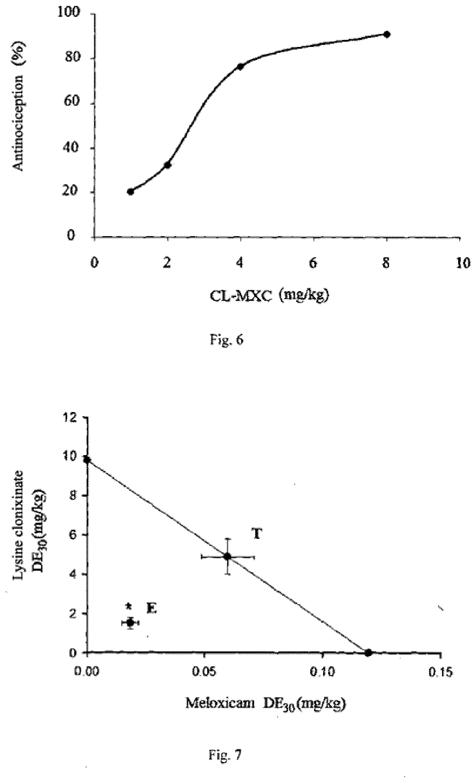

An anti-nociceptive dose-response curve with different doses of the combination was made using the ratio MXC:CL (1:33). On the dose-response curve in Figure 6 it can be seen that the maximal anti-nociceptive effect obtained using an 8.6 mg/kg dose of CL-MXC was of 91 %, which means that it is possible to surpass the analgesic ceiling effect observed on individual drugs. The experimental $DE_{30}$ obtained was of $1.53 \pm 0.08$ mg/kg.

The difference between the theoretical $DE_{30}$ ($4.96 \pm 0.92$ mg/kg) and the experimental $DE_{30}$ ($1.53 \pm 0.08$ mg/kg) was calculated using a Student's t test finding a significant difference between both (p<0.05). This means that the intraperitoneal co-administration of MXC and CL in this model of nociception generates a discrete synergistic interaction. The magnitude of the interaction was calculated based on the following formula:

$$\gamma = \frac{dosis\ of\ drug\ 1\ in\ the\ DE30\ of\ the\ combination}{individual\ DE30\ of\ drug\ 1} +$$

$$\frac{dosis\ of\ drug\ 2\ en\ the\ DE30\ of\ the\ combination}{individual\ DE30\ of\ drug\ 2}$$

Wherein $\gamma$ is the drug interaction index, individual $DE_{30}$ is the dose of drug 1 which has the same effect (30% anti-nociception) as drug 2 of the combination, and the combination's $DE_{30}$ is the dose which has the same anti-nociceptive effect value of 30%. The interaction index describes the experimental $DE_{30}$ as a fraction of the theoretical $DE_{30}$; values close to 1 indicate an additive interaction, whereas values higher than 1 involve antagonistic interaction and values lower than 1 indicate synergistic interaction between the drugs after intraperitoneal administration. That is, after an intraperitoneal co-administration of MXC and CL, the interaction index calculated was $0.308 \pm 0.079$, which suggests that the analgesic effect resulting from the combination of MXC:CL in a ratio of (1:33) is about 3 times higher than the one obtained when the drugs are individually administered.

Figure 7 shows the isobologram of the systematic interaction between MXC and CL in the anti-nociceptive effect in the formalin inflammatory pain test. The values of the CL and MXC doses are represented on one of each axis. The line connecting the points that represent the $DE_{30}$ of each drug is called isobole or line of additivity, and this line contains all possible combinations of the two drugs that produce only a theoretical additivity or summation effect. Point T corresponds to the theoretical $DE_{30}$ for this study ($4.96 \pm 0.92$ mg/kg). Point E is the experimental $DE_{30}$ ($1.53 \pm 0.08$ mg/kg) of the combination and, in this case, it is below the isobole, indicating a synergistic interaction between MXC and CL (*p<0.05). On the contrary, if the experimental point had been determined as being above this line of additivity, that would mean that an antagonism when administering the two drugs had occurred, which was not the case.

*Anti-inflammatory efficacy of CL-MXC*

The ratio 1:33 (MXC:CL) was used in order to evaluate the anti-inflammatory effect of the CL-MXC combination in the rat model of carrageen-induced inflammation.

*Animals:* Male Wistar rats aged 10-12 weeks and weighing 180 and 220 g were used. The animals were kept in boxes with food and water *ad libitum* until the time of the experiment, and with 12 x 12 h light-dark cycles. All experiments were performed following the ethical guidelines for pain research in experimental animals (Zimmermann, 1983).

*Drugs and reagents:* Both meloxicam and lysine clonixinate were used as raw materials, provided by Farmacéuticos Rayere S.A (Benito Juárez, Mexico D.F.). The remaining reagents were reagent grade. The carrageen (lambda fraction) was purchased from Sigma Chemical Co. (St. Louis, MO) and the sterile 0.9% saline solution from commercial sources. The drugs administered individually and in combinations were dissolved in purified water (CL) or in still mineral water (MXC) and orally administered(1 ml/kg).

*Experimental model of carrageen to evaluate the anti-inflammatory response:* The carrageen model consists of the subcutaneous administration on the right lower foot-pad of the rat of 0.1 ml of a 1 % carrageen suspension in saline solution (0.9 %). The induced inflammation is measured by plethysmography.

The carrageen model is one of the most commonly used to develop oedemas in animal models, since it presents the following advantages:

- The offending substance (carrageen) is stable and the quantities needed for generating an inflammation pattern similar to the pattern of human rheumatoid arthritis have been standardised.
- It can be used as an acute, sub-acute or chronic model of inflammation.
- The evolution of inflammation is similar to that experienced by humans. It is highly reproducible and presents low variability between subjects.
- It is a model that enables a quantitative assessment of the caused inflammation, so that the conclusions are not subjective or indirect.

The generation of oedemas by means of carrageen has been widely studied. It has been proved that it leads to an increase in vascular permeability and damage to the connective tissue (Crunkhorn P and Meacock S, 1971; Ferreira S, Moncada S, Parsons M et al. 1974).

Groups of 6 rats were formed in order to verify the anti-inflammatory effect of the CL-MXC combination. The anti-inflammatory effect of the CL-MXC combination (MXC:CL 1:33.3; 0.04 mg/kg + 1.49 mg/kg by oral route) was evaluated, as well as the effect of 2-fold and 4-fold doses, compared with the original drugs at their highest doses in the combination and the control (carrier), in a multiple oral administration schedule (every 8/h) during a period of 48 h after the application of an inflammatory stimulus in rats.

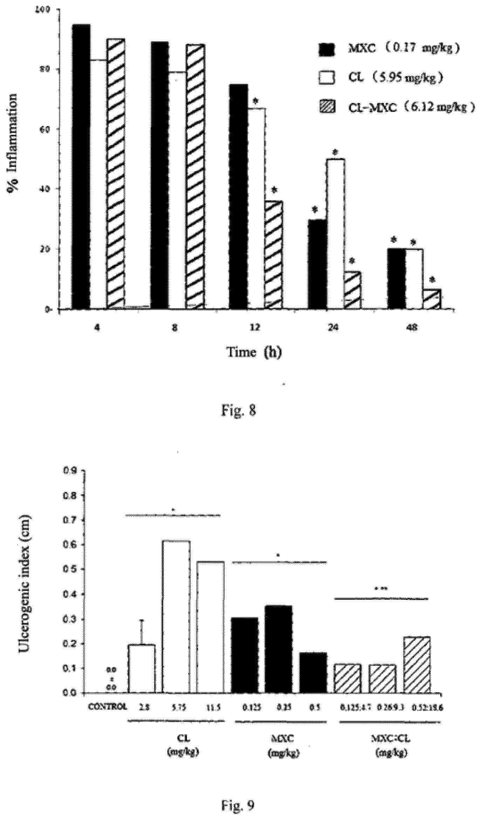

The percentage of inflammation is shown in Figure 8, determined at 4, 8, 12, 24 and 48 hours after the subcutaneous administration of carrageen in rats treated with repeated oral doses (every /8 h) of MXC (0.17 mg/kg), CL (5.95 mg/kg) individually or combined in a ratio of 1:33.3 in doses of 6.12 mg/kg. The drug administration was performed 8 hours after the injection of carrageen and the drugs were administered 20 min before beginning the determinations. The anti-inflammatory effect is noticeable after 12 hours in the Cl and CL-MXC groups (* $p < 0.05$ vs control). In the group treated with the CL-MXC combination, the percentage of inflammation decreased by 3 to 4 times compared to the groups treated with the individual drugs, which suggests that its anti-inflammatory effect is similar to that provided by doses of the individual drugs 3 to 4 times higher than those required. This could result in a more beneficial and safe clinical management for the treatment of conditions involving pain of inflammatory origin.

*Toxicological profile of CL-MXC*

For the purpose of determining the possible toxic effects of the CL-MXC combination, preclinical studies of the CL-MXC combination and of the individual drugs CL and MXC were performed using dosage levels equivalent to the maximal clinically allowed doses of the individual drugs (MXC: 0.25 and CL: 11.5 mg/kg/day by oral route). Experimental models were employed to determine the acute and sub-chronic gastrointestinal toxicity of the CL-MXC combination through ulcerogenicity tests. The motor and CNS toxicity were also evaluated after the acute and sub-chronical administration of the treatments in mice, as well as the effect of the continuous exposure to the CL-MXC combination based on some indicators of the liver function in rats. Finally, the lethality ($DL_{50}$) of the combination CL-MXC was determined so as to establish its safety profile. Figure 9 shows the ulcerogenic activity after the repeated administration (twice a day during 7 days) of the CL-MXC combination (1:33) at three concentration levels in comparison with the individual drugs CL and MXC at the maximal dosage levels (mg/kg), and the carrier administered control on mice in groups each one of 6. The UI was calculated as the total aggregated length of ulcers and/or irritation areas that the GI tissue presented. The statistical evaluation consisted of a chi-square goodness of fit test for the ordinal scale and of performing an analysis of variance, followed by Dunnett's test to evaluate the UI. It was concluded that significant changes occurred when $p < 0.05$. The results show that the CL-MXC combination presents the lowest UI values in comparison with the treatments with the individual drugs, including those using the highest dose of 0.5:18.6 mg/kg (MXC:CL). The groups *$p < 0.05$ vs. control; **$p < 0.05$ are indicated regarding the treatment with a higher ulcerogenic effect (CL 5.75 mg/kg).

As for the possible damage to the central nervous system and the autonomic nervous system, neither the acute (single dose) nor the sub-chronic (2 doses/day during 7 days) administration of the CL-MXC combination caused neurological nor vegetative changes at the two lower dosage levels. It was only observed that the administration of the highest dose tested was able to increase the alertness and the exploratory activity in less than 10 % of the tested animals. The liver enzyme profile did not present significant changes either. The values found in the groups treated with the CL-MXC recorded normal physiological levels. The lethal dose 50 (DL50) of the combination found in the lethality study was 1594.1 mg/kg, representing a dose more than 1000 times higher than the therapeutic doses when added (CL + MXC).

The evidence presented proved that the combination of lysine clonixinate and meloxicam enhances the analgesic and the anti-inflammatory effects that enable to formulate safe pharmaceutical compositions, containing less CL or MXC that when these drugs are individually used, and with a higher safety profile. These preparations are aimed at the treatment of moderate to severe acute pain and the consequent inflammatory processes, with the advantage of relieving pain with high efficacy.

**BIBLIOGRAPHY**

Barkin RL. Acetaminophen, aspirin, or ibuprofen in combination analgesic products. American Journal of Therapeutics. 8 (6): 433-442, 2001.

Blanco F, Guitian R, Moreno J, de Toro F, Galdo F. Effect of anti-inflammatory drugs on COX-1 and COX-2 activity in human articular chondrocytes. J Rheumatol. 26 (6): 1366-73, 1999.

Crunkhorn P y Meacock S. Mediators of the inflammation induced in the rat paw by carrageenin. Br J Pharmacol. 42 (3): 392-402,1971.

Dahl J, Rosenberg J, Dirkes W, Mogensen T, Kehlet H. Prevention of postoperative pain by balanced analgesia. Br J Anaesth. 64 (4): 518-20, 1990.

Dequeker J, Hawkey C, Kahan A, Steinbrück K, Alegre C, Baumelou E, Bégaud B, Isomäki H, Littlejohn G, Mau J, Papazoglou S. Improvement in gastrointestinal tolerability of the selective cyclo-oxygenase (COX)-2 inhibitor, meloxicam, compared with piroxicam: results of the safety and efficacy large-scale evaluation of COX inhibiting therapies (SELECT) trial in osteoarthritis. Br. J. Rheumatol. 37: 946-951, 1998.

Di Girolamo G, Farina M, Ribeiro M, Ogando D, Aisemberg J, de los Santos A, Marti M, Franchi A. Effects of Cyclooxygenase Inhibitor Pretreatment on Nitric Oxide production, nNOS and iNOS expression in rat cerebellum. Br. J. Pharmacol. 139: 1164-1170, 2003.

Dubois RW, Melmed GY, Henning JM, Bernal M. Risk of Upper Gastrointestinal Injury and Events in Patients Treated With Cyclooxygenase COX-1/COX-2 Nonsteroidal Anti-inflammatory Drugs (NSAIDs), COX-2 Selective NSAIDs, and Gastroprotective Cotherapy: An Appraisal of the Literature. J Clin Rheumatol. 10 (4): 178-189, 2004.

Dubuisson D., Dennis SG. The formalin test: a quantitative study of the analgesic effects of morphine, meperidine and brain stem stimulation in rats and cats. Pain. 4 (2): 161-174, 1977.

Engelhardt G, Homma D, Schlegel K, Utzmann R y Schnitzler C. Anti-inflammatory, analgesic, antipyretic and related properties of meloxicam, a new non-steroidal anti-inflammatory agent with favourable gastrointestinal tolerance. Inflammation Research, 44 (10): 423-433, 1995.

Ferrari P. Analgesic method containing Lysine 2-(2-methyl-3-chloro-anilino)-3-nicotinate. United States Patent 3,973,027, August 3, 1976.

Ferreira S, Moncada S, Parsons M y Vane J. Proceedings: the concomitant release of bradykinin and prostaglandin in the inflammatory response to carrageenin. Br J. Pharmacol. 52 (1): 108P-109P, 1974

Franchi A, Di Girolamo G, Farina M, Marti M. Acción Diferencial de los Antiinflamatorios No esteroides Sobre la Ciclooxigenasa y Lipooxigenasa de Vesicula Biliar Humana. Medicina (Buenos Aires). 60: 580-586, 2000.

Gates BJ, Nguyen TT, Setter SM, Davies NM. Meloxicam: a reappraisal of pharmacokinetics, efficacy and safety. Expert Opin Pharmacother. 6 (12): 2117-40, 2005.

Gronert K. Lipid autacoids and privileged injury responses. Molecular Interventions. 8: 28-35, 2008.

Hawkey C, Kahan A, Steinbruck K, Alegre C, Baumelou E, Bégaud B, Dequeker J, Isomäki H, Littlejohn G, Mau J, Papazoglou S. Gastrointestinal tolerability of meloxicam compared to diclofenaco in osteoarthritis patients. Br. J. Rheumatol. 37: 937-945, 1998.

Kelhet H, Wemer M, Perkins F. Balanced Analgesia: What is it and what are its advantages in postoperative pain? Drugs. 58: 793-797, 1999.

Kramer E, Sassetti B, Kaminker A, De Los Santos A, Martí M, Di Girolamo G. Effects of lysine clonixinate on platelet function. Comparison with other non-steroidal anti-inflammatory agents. Medicina (B Aires). 61 (3): 301-7, 2001.

Krymchantowski A, Barbosa J, Cheim C, Alves L. Oral lysine clonixinate in the acute treatment of migraine: a double-blind placebo-controlled study. Arq. Neuropsiquiatr. 59 (1): 46-49, 2001.

Latremoliere A, Woolf C. Central Sensitization: A Generator of Pain Hypersensitivity by Central Neural Plasticity. J Pain. 10 (9): 895-926, 2009.

Cyclooxygenase. Proc. Natl. Acad. Sci. USA. Pharmacology. 90: 11693-11697, 1994.

Ong C., Lirk P., Tan C. y Seymour R. An Evidence-Based Update on Nonsteroidal Anti-Inflammatory Drugs. Clinical Medicine & Research. 1: 19-34, 2007.

Orti E., Coirini H. y Pico J. Site-specific effects of the nonsteroidal anti-inflammatory drug lysine clonixinato on rat brain opioid receptors. Pharmacology. 58: 190-199, 1998.

Pallapies D, Salinger A, Meyer zum Gottesberge A, Atkins DJ, Rohleder G, Nagyivanyi P, Peskar BA. Effects of lysine clonixinate and ketorolac tromethamine on prostanoid release from various rat organs incubated ex vivo. Life Sci. 57 (2): 83-9, 1995.

Samad T., Sapirstein A. y Woolf C. Prostanoids and pain: Unraveling mechanisms and revealing therapeutic targets. Trends Mol Med. 8: 390-396, 2002.

Sagripanti A, Ferdeghini M, Pinori E, Polloni A, Bianchi R y Materazzi F. Platelet activation in outpatients undergoing esophagogastroduodenoscopy. J Nucl Med Allied Sci. 33: 22-25, 1989.

Seibert K, Zhang Y, Leahy K, Hauser S, Masferrer J, Perkins W, Lee L, Isakson P. Pharmacological and biochemical demonstration of the role of cyclooxygenase 2 in inflammation and pain. Proc Natl Acad Sci USA. 6: 91 (25): 12013-7, 1994.

Tallarida R. Drug synergism and dose-effect data analysis. New York. Chapman & Hall/CRC. pp. 1-72, 2000.

Tavares I. A. The effects of meloxicam, indomethacin or NS-398 on eicosanoid synthesis by fresh human gastric mucosa. Aliment Pharmacol Ther. 14: 795-799, 2000.

Tilley S., Coffman T. y Koller B. Mixed messages: Modulation of inflammation and immune responses by prostaglandins and thromboxanes. J Clin Invest. 108: 15-23, 2001.

Vandana R. Autacoids: Angiotensin, plasma kinins. Pharmacology, Publ. Maulana Azad Medical College & associated Hospitals, New Delhi, pp 1-35, 2007.

Winter C., Risley E., Nuss G. Carrageenin-induced edema in hind paw of the rat as an assay for antiiflammatory drugs. Proc Soc Exp Biol Med. 111: 544-7, 1962.

World Health Organization. Cancer Pain Relief. Albany, NY: WHO Publications Center; 1986.

Zeilhofer H. Prostanoids in nociception and pain. Biochem Pharmacol. 73:165-174, 2007:

Zimmermann M. Ethical guidelines for investigations on experimental pain in conscious animals. Pain. 16: 109-110, 1983.

## Claims

1. An analgesic pharmaceutical composition comprising a combination of meloxicam or a pharmaceutically acceptable salt thereof, in all its crystalline forms, and lysine clonixinate, in all its crystalline forms, in a weight ratio of 1:1 to 1:50 respectively.

2. A pharmaceutical composition according to claim 1, **characterised in that** the ratio of meloxicam: lysine clonixinate is 1:33 and 1:50.

3. A pharmaceutical composition according to claims 1 or 2 further comprising a pharmaceutically acceptable carrier.

4. A pharmaceutical composition according to claims 1 to 3 in the form of an injectable solution.

5. A pharmaceutical composition according to claims 1 to 3 in the form of tablets, capsules, oral solutions, oral suspensions, gels, ointments and suppositories.

6. A pharmaceutical composition according to claims 1 to 5 for use in analgesic therapy.

7. A pharmaceutical composition according to claims 1 to 5 for use in anti-inflammatory therapy.

## Patentansprüche

1. Analgetische pharmazeutische Zusammensetzung, umfassend eine Kombination aus Meloxicam oder einem pharmazeutisch verträglichen Salz davon, in all seinen kristallinen Formen, und Lysinclonixinat, in all seinen kristallinen Formen, in einem Gewichtsverhältnis von jeweils 1:1 bis 1:50.

2. Pharmazeutische Zusammensetzung nach Anspruch 1, **dadurch gekennzeichnet, dass** das Verhältnis von Meloxicam: Lysinclonixinat 1:33 und 1:50 beträgt.

3. Pharmazeutische Zusammensetzung nach Anspruch 1 oder 2, ferner umfassend einen pharmazeutisch verträglichen Träger.

4. Pharmazeutische Zusammensetzung nach Anspruch 1 bis 3 in Form einer injizierbaren Lösung.

5. Pharmazeutische Zusammensetzung nach Anspruch 1 bis 3 in Form von Tabletten, Kapseln, oralen Lösungen, oralen Suspensionen, Gelen, Salben und Zäpfchen.

6. Pharmazeutische Zusammensetzung nach Anspruch 1 bis 5 zur Verwendung in der analgetischen Therapie.

7. Pharmazeutische Zusammensetzung nach Anspruch 1 bis 5 zur Verwendung in der entzündungshemmenden Therapie.

## Revendications

1. Composition pharmaceutique analgésique comprenant une combinaison de méloxicam ou un sel pharmaceutiquement acceptable de celui-ci, sous toutes ses formes crystallines, et de clonixinate de lysine, dans toutes ses formes crystallines, dans un rapport en poids de 1:1 à 1:50 respectivement.

**2.** Composition pharmaceutique selon la revendication 1, **caractérisée en ce que** le rapport de méloxicam : clonixinate de lysine est de 1:33 et 1:50.

**3.** Composition pharmaceutique selon les revendications 1 ou 2 comprenant en outre un excipient pharmaceutiquement acceptable.

**4.** Composition pharmaceutique selon les revendications 1 à 3 sous la forme d'une solution injectable.

**5.** Composition pharmaceutique selon les revendications 1 à 3 sous la forme de comprimés, capsules, solutions orales, suspensions orales, gels, pommades et suppositoires.

**6.** Composition pharmaceutique selon les revendications 1 à 5 pour son utilisation dans la thérapie analgésique.

**7.** Composition pharmaceutique selon les revendications 1 à 5 pour son utilisation dans la thérapie antiinflammatoire.

CL

Fig.1

Meloxicam

Fig.2

Fig.3

Formalin test phase II

Fig.4

Formalin test phase II

Fig. 5 .

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007281927 A1
- US 3973027 A
- EP 0492747 A2
- WO 2007083985 A
- WO 2007148950 A
- WO 2012176155 A1
- US 2004229038 A1

**Non-patent literature cited in the description**

- **BARKIN RL.** Acetaminophen, aspirin, or ibuprofen in combination analgesic products. *American Journal of Therapeutics,* 2001, vol. 8 (6), 433-442
- **BLANCO F ; GUITIAN R ; MORENO J ; DE TORO F ; GALDO F.** Effect of anti-inflammatory drugs on COX-1 and COX-2 activity in human articular chondrocytes. *J Rheumatol.,* 1999, vol. 26 (6), 1366-73
- **CRUNKHORN P Y MEACOCK S.** Mediators of the inflammation induced in the rat paw by carrageenin. *Br J Pharmacol.,* 1971, vol. 42 (3), 392-402
- **DAHL J ; ROSENBERG J ; DIRKES W ; MOGENSEN T ; KEHLET H.** Prevention of postoperative pain by balanced analgesia. *Br J Anaesth.,* 1990, vol. 64 (4), 518-20
- **DEQUEKER J ; HAWKEY C ; KAHAN A ; STEIN-BRÜCK K ; ALEGRE C ; BAUMELOU E ; BÉGAUD B ; ISOMÄKI H ; LITTLEJOHN G ; MAU J.** Improvement in gastrointestinal tolerability of the selective cyclo-oxygenase (COX)-2 inhibitor, meloxicam, compared with piroxicam: results of the safety and efficacy large-scale evaluation of COX inhibiting therapies (SELECT) trial in osteoarthritis. *Br. J. Rheumatol.,* 1998, vol. 37, 946-951
- **DI GIROLAMO G ; FARINA M ; RIBEIRO M ; OGANDO D ; AISEMBERG J ; DE LOS SANTOS A ; MARTI M ; FRANCHI A.** Effects of Cyclooxygenase Inhibitor Pretreatment on Nitric Oxide production, nNOS and iNOS expression in rat cerebellum. *Br. J. Pharmacol.,* 2003, vol. 139, 1164-1170
- **DUBOIS RW ; MELMED GY ; HENNING JM ; BERNAL M.** Risk of Upper Gastrointestinal Injury and Events in Patients Treated With Cyclooxygenase COX-1/COX-2 Nonsteroidal Anti-inflammatory Drugs (NSAIDs), COX-2 Selective NSAIDs, and Gastroprotective Cotherapy: An Appraisal of the Literature. *J Clin Rheumatol.,* 2004, vol. 10 (4), 178-189
- **DUBUISSON D. ; DENNIS SG.** The formalin test: a quantitative study of the analgesic effects of morphine, meperidine and brain stem stimulation in rats and cats. *Pain.,* 1977, vol. 4 (2), 161-174
- **ENGELHARDT G ; HOMMA D ; SCHLEGEL K ; UTZMANN R Y SCHNITZLER C.** Anti-inflammatory, analgesic, antipyretic and related properties of meloxicam, a new non-steroidal anti-inflammatory agent with favourable gastrointestinal tolerance. *Inflammation Research,* 1995, vol. 44 (10), 423-433
- **FERREIRA S ; MONCADA S ; PARSONS M Y VANE J.** Proceedings: the concomitant release of bradykinin and prostaglandin in the inflammatory response to carrageenin. *Br J. Pharmacol.,* 1974, vol. 52 (1), 108, , 109P
- **FRANCHI A ; DI GIROLAMO G ; FARINA M ; MARTI M.** Acción Diferencial de los Antiinflamatorios No esteroides Sobre la Ciclooxigenasa y Lipooxigenasa de Vesicula Biliar Humana. *Medicina (Buenos Aires),* 2000, vol. 60, 580-586
- **GATES BJ ; NGUYEN TT ; SETTER SM ; DAVIES NM.** Meloxicam: a reappraisal of pharmacokinetics, efficacy and safety. *Expert Opin Pharmacother.,* 2005, vol. 6 (12), 2117-40
- **GRONERT K.** Lipid autacoids and privileged injury responses. *Molecular Interventions,* 2008, vol. 8, 28-35
- **HAWKEY C ; KAHAN A ; STEINBRUCK K ; ALE-GRE C ; BAUMELOU E ; BÉGAUD B ; DEQUEKER J ; ISOMÄKI H ; LITTLEJOHN G ; MAU J.** Gastrointestinal tolerability of meloxicam compared to diclofenaco in osteoarthritis patients. *Br. J. Rheumatol.,* 1998, vol. 37, 937-945
- **KELHET H ; WEMER M ; PERKINS F.** Balanced Analgesia: What is it and what are its advantages in postoperative pain?. *Drugs.,* 1999, vol. 58, 793-797
- **KRAMER E ; SASSETTI B ; KAMINKER A ; DE LOS SANTOS A ; MARTÍ M ; DI GIROLAMO G.** Effects of lysine clonixinate on platelet function. Comparison with other non-steroidal anti-inflammatory agents. *Medicina (B Aires),* 2001, vol. 61 (3), 301-7

- **KRYMCHANTOWSKI A ; BARBOSA J ; CHEIM C ; ALVES L.** Oral lysine clonixinate in the acute treatment of migraine: a double-blind placebo-controlled study. *Arq. Neuropsiquiatr.,* 2001, vol. 59 (1), 46-49
- **LATREMOLIERE A ; WOOLF C.** Central Sensitization: A Generator of Pain Hypersensitivity by Central Neural Plasticity. *J Pain.,* 2009, vol. 10 (9), 895-926
- Cyclooxygenase. *Proc. Natl. Acad. Sci. USA. Pharmacology.,* 1994, vol. 90, 11693-11697
- **ONG C. ; LIRK P. ; TAN C. Y SEYMOUR R.** An Evidence-Based Update on Nonsteroidal Anti-Inflammatory Drugs. *Clinical Medicine & Research.,* 2007, vol. 1, 19-34
- **ORTI E. ; COIRINI H. Y PICO J.** Site-specific effects of the nonsteroidal anti-inflammatory drug lysine clonixinato on rat brain opioid receptors. *Pharmacology,* 1998, vol. 58, 190-199
- **PALLAPIES D ; SALINGER A ; MEYER ZUM GOTTESBERGE A ; ATKINS DJ ; ROHLEDER G ; NAGYIVANYI P ; PESKAR BA.** Effects of lysine clonixinate and ketorolac tromethamine on prostanoid release from various rat organs incubated ex vivo. *Life Sci.,* 1995, vol. 57 (2), 83-9
- **SAMAD T. ; SAPIRSTEIN A. Y WOOLF C.** Prostanoids and pain: Unraveling mechanisms and revealing therapeutic targets. *Trends Mol Med.,* 2002, vol. 8, 390-396
- **SAGRIPANTI A ; FERDEGHINI M ; PINORI E ; POLLONI A ; BIANCHI R Y MATERAZZI F.** Platelet activation in outpatients undergoing esophagogastroduodenoscopy. *J Nucl Med Allied Sci.,* 1989, vol. 33, 22-25
- **SEIBERT K ; ZHANG Y ; LEAHY K ; HAUSER S ; MASFERRER J ; PERKINS W ; LEE L ; ISAKSON P.** Pharmacological and biochemical demonstration of the role of cyclooxygenase 2 in inflammation and pain. *Proc Natl Acad Sci USA.,* 1994, vol. 6: 91 (25), 12013-7
- **TALLARIDA R.** Drug synergism and dose-effect data analysis. Chapman & Hall/CRC, 2000, 1-72
- **TAVARES I.** A. The effects of meloxicam, indomethacin or NS-398 on eicosanoid synthesis by fresh human gastric mucosa. *Aliment Pharmacol Ther.,* 2000, vol. 14, 795-799
- **TILLEY S. ; COFFMAN T. Y KOLLER B.** Mixed messages: Modulation of inflammation and immune responses by prostaglandins and thromboxanes. *J Clin Invest.,* 2001, vol. 108, 15-23
- Autacoids: Angiotensin, plasma kinins. **VANDANA R.** Pharmacology. Maulana Azad Medical College & associated Hospitals, 2007, 1-35
- **WINTER C. ; RISLEY E. ; NUSS G.** Carrageenin-induced edema in hind paw of the rat as an assay for antiiflammatory drugs. *Proc Soc Exp Biol Med.,* 1962, vol. 111, 544-7
- World Health Organization. Cancer Pain Relief. WHO Publications Center, 1986
- **ZEILHOFER H.** Prostanoids in nociception and pain. *Biochem Pharmacol.,* 2007, vol. 73, 165-174
- **ZIMMERMANN M.** Ethical guidelines for investigations on experimental pain in conscious animals. *Pain,* 1983, vol. 16, 109-110